Europäisches Patentamt

European Patent Office

Office européen des brevets

(11) Veröffentlichungsnummer: **0 064 277**
**B1**

# (12) EUROPÄISCHE PATENTSCHRIFT

(45) Veröffentlichungstag der Patentschrift:
19.03.86

(21) Anmeldenummer: 82103647.2

(22) Anmeldetag: 29.04.82

(51) Int. Cl.⁴: **A 61 F 2/18, A 61 L 17/00**

(54) Gehörgangswand-Prothese in Form eines Hohlkörpers und Verfahren zu ihrer Herstellung.

**Verbunden mit 82901402.6/77370 (europäische Anmeldenummer/Veröffentlichungsnummer) durch Entscheidung vom 30.01.84.**

(30) Priorität: 29.04.81 DE 3117025
26.03.82 DE 3211212

(43) Veröffentlichungstag der Anmeldung:
10.11.82 Patentblatt 82/45

(45) Bekanntmachung des Hinweises auf die Patenterteilung:
19.03.86 Patentblatt 86/12

(84) Benannte Vertragsstaaten:
FR GB IT NL

(56) Entgegenhaltungen:
WO - A - 82/01127
DE - B - 2 451 969
FR - A - 2 243 915
FR - A - 2 327 758
US - A - 3 919 723
US - A - 3 922 155
US - A - 4 052 754
US - A - 4 131 597
US - A - 4 169 292

(73) Patentinhaber: ERNST LEITZ WETZLAR GMBH,
Ernst-Leitz-Strasse 30 Postfach 20 20,
D-6330 Wetzlar 1 (DE)

(72) Erfinder: Reck, Ralf, Südring 189,
D-6500 Mainz-Bretzenheim (DE)
Erfinder: Brömer, Heinz, Hundsrück 7, D-6330 Wetzlar (DE)
Erfinder: Deutscher, Klaus, Lerchenweg 20,
D-6330 Wetzlar (DE)
Erfinder: Franek, Henning, Mühlenkopfstrasse 5,
D-6333 Braunfels-Tiefenbach (DE)

LIBER, STOCKHOLM 1986

**Beschreibung**

Die Erfindung betrifft eine Prothese in Form eines Hohlkörpers für den totalen bzw. partiellen Ersatz der hinteren Gehörgangswand Jowie ein Verfahren zu ihrer Herstellung.

Aufgrund pathologischer Gegebenheiten kann es erforderlich werden, einen Teil der hinteren Gehörgangswand bzw. sogar den gesamten hinteren Gehörgangstrakt durch ein Dauer-Implantat zu ersetzen.

Aus der US-A-4 169 292 ist eine kombinierte Mittelohrund Gehörgangswandprothese bekannt. Sie weist eine geschlossene Zylindermantelfläche auf. Zusätzlich ist in der Ebene einer der Deckflächen ein flacher Ring mit Innen- und Aufsatz-Segmenten angebracht, die die Funktionen der Gehörknöchelchenkette übernehmen sollen. Das Gesamtgebilde besteht aus lediglich biokompatiblem Kunststoffmaterial mit mikroporiger Oberfläche.

Für den Oto-Chirurgen besteht die Aufgabe darin, eine den tatsächlichen anatomischen Verhältnissen analoge Prothesenform vorzusehen, das Materlalproblem für die vorggsehene Prothese in optimaler Weise zu lösen und an der Prothese Mittel für das anatomisch-geometrisch exakte Zurichten und Positionieren der Prothese am Implantationsort vorzusehen. Der Erfindung liegt weiterhin die Aufgabe zugrunde, diejenigen Oberflächenteilbereiche der vorliegenden Prothese die nicht für den eigentlichen Verbund mit dem Knochen (Verwachsungszone: Implantat/knöchernes Hartgewebe) vorgesehen sind, derart auszubilden, daß sie biochemisch vollkommen resistent und bioaktiv sind. Darüber hinaus besteht die Aufgabe darin, ein Verfahren zur nachträglichen Bioinaktivierung von ausgewählten Teilbereichen an sich bioaktiver Prothesen bzw. Prothesenteile anzugeben.

Die Aufgabe wird bei einer Prothese der eingangs genannten Art erfindungsgemäß dadurch gelöst, daß sie die Form einer nicht geschlossenen, hohlzylindrischen Manschette mit mindestens in ihren beiden Grundflächenbereichen vorhandenen unterschiedlichen Aufweitungen nach Art eines Hyperboloids mit vorzugsweise ellipsenförmigem Querschnitt aufweist und daß sie aus bioaktivem Material besteht. Nach einer besonderen Ausführungsform weist ihre Innenwandung ein räumliches Koordinaten-Raster auf. Dabei kann das Raster aus zwei sich kreuzenden Scharen von Linien bestehen, wobei die eine der beiden Scharen aus einer Vielzahl von hyperbelförmigen, im wesentlichen in Richtung der Achse der Manschette verlaufenden Linien und die andere der beiden Scharen aus einer Vielzahl von ellipsenförmigen im wesentlichen senkrecht zur Achse der Manschette verlaufenden Lienien besteht. Die Linien des Koordinaten-Rasters können als rillenförmige Vertiefungen von beliebiger Querschnittsform - beispielsweise dreieckig, viereckig, abgerundet, usw. -

ausgebildet sein. Auch ist es möglich, die Linien des Koordinaten-Rasters als wulstartige Erhöhungen auszubilden. Nach einer weiteren Ausführungsform der vorliegenden Erfindung können anstelle kontinuierlicher Linien diskontinuierliche periodisch angeordnete Raster, beispielsweise Grübchen oder Noppen, vorgesehen sein. Dabei ist es zweckmäßig, daß die positiven Verkörperungen des Rasters aus demselben Material wie die Prothese selbst bestehen. Die mittlere Maschenweite des Koordinaten-Rasters beträgt zwischen 1 und 2 mm. Mit Vorteil ist die Länge des Manschetten-Ausschnittes größer als dessen mittlere Peripher-Distanz und die kürzeste aller Hauptachsen derjenigen Innen-Ellipsen, die durch Schnitte senkrecht zur Achse der Manschette erzeugt werden, ist länger als die mittlere Peripher-Distanz des Manschetten-Ausschnittes. Die Dimensionierung kann so getroffen sein, daß die Länge der Manschette zu den beiden Mantel-Schnittstellen hin, an welchen der Manschetten-Ausschnitt herausgetrennt ist kontinuierlich größer wird und dort ihr Maximum erreicht während ihr Minimum bei dem dem Ausschnitt gegenüberliegenden Mantelbereich erreicht wird. Nach einer speziellen Ausführungsform der vorliegenden Erfindung beträgt die Wanddicke der Manschette zwischen 0,5 und 2,0 mm die mittlere Länge der Manschette zwischen 12 und 25 mm die mittlere Peripher-Distanz des Manschetten-Ausschnittes zwischen 7 und 14 mm, die mittlere Länge der Ellipsen-Hauptachsen zwischen 10 und 17 mm die mittlere Länge der Ellipsen-Nebenachsen zwischen 5 und 8 mm und das positive bzw. negative Raster weist eine mittlere Höhe bzw. Tiefe zwischen 0,2 und 1,0 mm auf.

Das verwendete bioaktive Material ist vorzugsweise Bio-Glaskeramik. Es ist indes auch möglich, andere Biomaterialien, wie bioaktives Verbundmaterial oder apatithaltige Sinterprodukte, vorzusehen. Schließlich ist es auch möglich, daß die vorgeschlagene Prothese aus einem Kernmaterial besteht, das eine Beschichtung mit bioaktiven Substanzen aufweist. Nach einer weiteren Ausgestaltung der Erfindung kann die Gehörgangswand-Prothese auch lediglich aus einem Teil der Gesamtmanschette, insbesondere aus einem Manschettenmantel-Abschnitt oder einem Teil davon, bestehen.

Die Aufgabe wird bei einer Prothese der eingangs genannten Art weiterhin dadurch gelöst, daß sie zusätzlich in mindestens einem Oberflächen-Teilbereich unlösliches bioinertes Material enthält bzw. partiell eine Beschichtung aus unlöslichem bioinerten Material aufweist. Dabei kann die Beschichtung aus mindestens einer additiv aufgebrachten Schutzschicht mit einer Dicke zwischen 0,25 und 10 µm oder aus mindestens einer subtraktiv erzeugten Konversionsschicht mit einer Dicke zwischen 0,25 und 5 µm bestehen. Es ist auch möglich daß die Beschichtung aus mindestens einer prothesenkernseitig erzeugten Konversionsschicht und mindestens einer auf dieser aufliegenden, additiv aufgebrachten Schutzschicht besteht. Mit Vorteil besteht zumindest die Innenwand und/oder die Außenwand der Manschette aus unlöslichem bioinerten Material. Es ist indes auch möglich, daß zusätzlich die Längsseiten der Manschette aus unlöslichem bioinerten Material bestehen. Das bioinerte Material bzw. die bioinerte Schicht kann aus mindestens einer der folgenden Substanzen bestehen: Metalle, wie Gold, Platin, Titan, sowie Metalllegierungen; Kohlenstoff in geeigneten Modifikationen, wie pyrolytischer Kohlenstoff (Graphit); Kohlenstoff-verbindungen, wie Siliziumkarbid (SiC), Titankarbid (TiC), Borkarbid ($B_4C$); Sonderkeramische Werkstoffe, wie hexagonales Bornitrid (BN), Titannitrid (TiN) Siliciumnitrid ($Si_3N_4$); teilkristalline anorganische Verbundsysteme, wie Emails; anorganische Einkomponenten- (z.B. Kieselglas) oder Mehrkomponenten-Gläser; Oxide, wie Titandioxid ($TiO_2$), Zirkondioxid ($ZrO_2$) und Aluminiumoxid ($Al_2O_3$). Nach einer vorteilhaften Ausführungsform besteht das bioinerte Material bzw. die bioinerte Schicht aus einer(m) apatitfreien Residual-Bioglaskeramik bzw. -Biokeramik bzw. -Bioglas, welche(s) gegebenenfalls zusätzlich eine Silan-Schicht aufweist.

Nach dem erfindungsgemäßen Verfahren werden die zu behandelnden Teilbereiche der Manschette unter gezielter Stoffzufuhr einer Oberflächen-Nachbehandlung zur Erzeugung mindestens einer additiv aufgebrachten, dauerhaft festsitzenden, in vivo als biochemische Sperrschicht wirkenden, bioinerten Schutzschicht unterworfen. Es ist aber ebenso möglich, ein Beschichtungsverfahren vorzusehen, bei dem die betreffenden Teilbereiche der Manschette bzw. des Manschettenteils unter gezielter Stoffabfuhr bzw. Stoffaustausch einer chemischen Nachbehandlung zur Erzeugung mindestens einer subtraktiv erhaltenen, permanent festhaftenden, in vivo als biochemische Sperrschicht wirkenden, bioinerten Konversions-(Auslaugungs-)Schicht interworfen werden. Die additive Schutzschicht kann vorzugsweise durch mindestens einen der folgenden-Verfahrensschritte aufgebracht werden: Galvanisieren oder Bedampfen; Aufsputtern; Abscheiden aus organischen Lösungen oder Aufdampfen im Vakuum; Tauchen, Sprühen oder Aufstreuen mit nachfolgender thermischer Behandlung; Eintauchen in Wasserglas mit anschließendem Aufheizen auf ca. 400°C bzw. Tauchglasieren aus ein- oder mehrkomponentigen Schmelzgemengen; Simultan-Aufdampfen oder Aufdampfen der Metalle mit nachfolgender Oxidierungsbehandlung.

Die subtraktive Schutzschicht kann gemäß der vorliegenden Erfindung erhalten werden, indem die zu beschichtende Mantelfläche mit wässrigen, sauren Lösungen oder mit wässrigen Salzlösungen mit Normalitäten zwischen 0 001 und 0,1 zwischen 5 Minuten und 3 Stunden bei Temperaturen zwischen 20 bis 100°C behandelt wird. Dabei kann als wässrige, saure Lösung 0,1 - 0,001 normale Salzsäure (HC1) oder als wässrige Salzlösung 0,001 bis 0,25 normale Standard-Azetat-Puffer-Lösung verwendet werden. Schließlich ist es möglich, die beschichteten Teile der Prothese anschließend einer thermischen Versiegelungs- und/oder Silanisierungsbehandlung zu unterwerfen.

In den Zeichnungen sind Ausführungsbeispiele der Erfindung schematisch dargestellt. Es zeigen:

Fig. 1: eine perspektivische Darstellung einer Total-Gehörgangswand-Prothese ("Manschette");

Fig. 2a: eine der Fig. 1 prinzipiell analoge Darstellung, jedoch mit einer graphischen Hervorhebung des Manschetten-Ausschnittes;

Fig. 2b: einen Schnitt durch das in Fig. 2a Dargestellte längs der Linie A-A;

Fig. 3: eine der Fig. 1 prinzipiell analoge Darstellung, jedoch mit einer graphischen Hervorhebung eines Teils eines Manschetten-Abschnittes (Partial-Gehörgangswand-Prothese);

Fig. 4a: eine der Fig. 1 analoge Darstellung (-das Innenwand-Linienraster wurde aus Gründen einer besseren Übersichtlichkeit nicht dargestellt -), bei der jedoch die Innen- und die Außenwand eine additive Schutzschicht aufweisen;

Fig. 4b: einen Schnitt durch das in Fig. 4a Dargestellte längs der Linie A-A;

Fig. 5a: eine der Fig. 1 entsprechende Darstellung jedoch mit zusätzlicher additiven Beschichtung auch der beiden Längsseiten der Manschette;

Fig. 5b: einen Schnitt durch das in Fig. 5a Dargestellte längs der Linie A-A.

Fig 6: das in Fig. 4b Dargestellte, jedoch mit einer subtraktiven Schicht;

Fig. 7: das in Fig. 6 Dargestellte, jedoch mit zusätzlicher subtraktiven Beschichtung auch der beiden Längsseiten der Manschette.

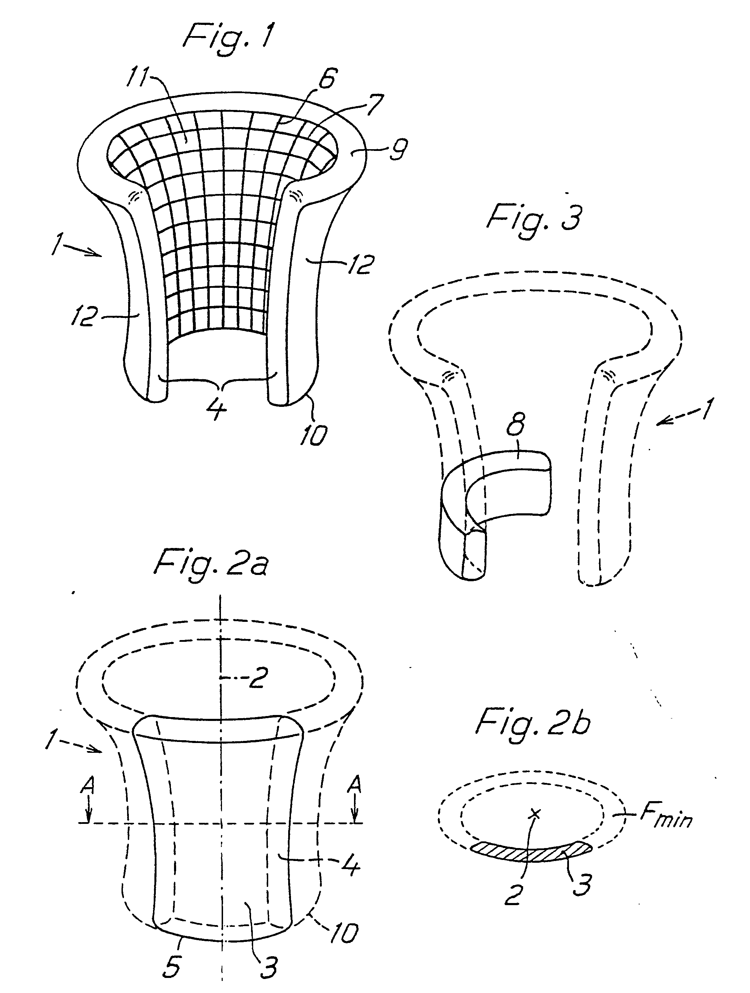

In Fig. 1 ist eine Total-Gehörgangswand-Prothese gemäß vorliegender Erfindung in perspektivischer, vergrößer-ter Form wiedergegeben. Zur Beschreibung der komplizierten geometrischen Raumform wird zunächst von den schematischen Darstellungen der Fig. 2a und 2b ausgegangen.

Stellt man sich in Fig. 2a auch den vorderen Ausschnitt in gestrichelter Linienführung vor, so ergibt sich eine geschlossene, hohlzylindrische Manschette mit von einer Kreisform abweichenden Querschnittsfläche. Wird eine Vielzahl von Schnitten, die auf der Manschetten-Achse 2 senkrecht stehen, durch die Manschette gelegt so weisen die Flächen unmittelbar benachbarter Schnitte unterschiedliche Flächeninhalte auf. In Fig. 2b ist die Fläche mitminimalem Flächeninhalt $F_{min}$ dargestellt, die durch einen Schnitt längs der Linie A-A in Fig. 2a erzeugt wurde.

Wie Fig. 2a zeigt, ist die geschlossene Manschette in ihrem oberen Bereich - also in Richtung zum oberen Manschettenrand 9 - stärker aufgeweitet, als in ihrem unteren Bereich. Die Schnittflächen, auf denen die Manschetten-Achse 2 jeweils senkrecht steht muß natürlich im streng mathematischen Sinne keine Ellipse - wie es in Fig. 2b idealisiert dargestellt istsein; vielmehr sind auch ähnliche geometrische Linienführungen möglich beispielsweise "deformierte" Kreise oder Ellipsen, schwach eiförmige Konturen oder ähnliche, kombinierte, geschlossene Gebilde. Charakteristisches Merkmal der geometrischen Ausgangsform ist jedoch die im Mittelbereich der Manschette vorhandene leichte Taillierung und die in beiden Endbereichen unterschiedlich starke Aufweitung.

Ausgehend von dieser geschlossenen, hyperboloidischen Hohlform gelangt man zur endgültigen erfindungsgemäßen Prothesenform für ein Totalimplantat, indem man längs der Mantelfläche in im wesentlichen axialer Richtung zwei "Schnitte" ausführt und den auf diese Weise erhaltenen Manschetten-Ausschnitt 3 entfernt, so daß die in Fig. 1 gezeigte offene Manschettenform erhalten wird.

Wie aus Fig. 2a zu erkennen ist, sind die (axialen) Längsseiten 4 der Manschette 1 bzw. des Manschetten-Ausschnittes 3 größer als dessen mittlere Peripher-Distanz 5. Die minimale Peripher-Distanz ist aus dem schraffierten Teil der Fig. 2b ersichtlich. Die maximale Peripher-Distanz befindet sich im oberen Manschettenbereich, der am stärksten aufgetweitet ist.

Legt man an die in Fig. 2a gestrichelt angedeutete Manschette, und zwar in ihrem oberen wie in ihrem unteren Abschlußbereich, je eine Ebene, so daß sie auf dem jeweils zugeordneten Manschettenrand 9 bzw. 10 aufliegt, so steht die Manschetten-Achse 2 auf diesen gedachten Ebenen nicht senkrecht. Beide Ebenen verlaufen - vorzugsweise - nicht genau parallel zueinander. Ihr Abstand ist im Bereich des Manschetten-Ausschnittes 3 größer als im gegenüberliegenden (in der Fig. 1: hinteren) Manschettenbereich. Daraus resultiert eine endgültige Manschetten-Form, die in ihrem vorderen unteren Teil im Ausschnitts-Bereich hemdkragenspitzenförmige Ausläufer aufweist.

Die Wandstärke der Manschette 1 ist im wesentlichen konstant. Es sind indes auch geringfügige Aufwulstungen bzw. Verdickungen in ihren Abschlußbereichen möglich.

Ein zweckmäßiges Merkmal der vorgeschlagenen Gehörgangswand-Prothese besteht darin, daß sie - vorzugsweise über den gesamten Bereich ihrer Innenwandung 11 - ein Raster aufweist, das aus zwei Scharen sich kreuzender Linien 6 bzw. 7 besteht. Im dargestellten Fall verlaufen die Linien 6 der ersten Schar hyperbelförmig in axialer Richtung und die Linien 7 der zweiten Schar ellipsenförmig in peripherer Richtung, so daß sich die Linien beider Systeme etwa unter einem Winkel von 90° schneiden. Wenngleich in der Fig. 1 aus Gründen einer zeichnerischen Vereinfachung lediglich Linien dargestellt sind, so handelt es sich doch in Wirklichkeit um reliefartige, dreidimensionale Kennungen. Es können Ausnehmungen in der Manschetten-Innenseite 11, beispielsweise Rillen oder Furchen, sein. Der Querschnitt dieser vorzugsweise gleichförmigen Vertiefungen kann rechteckig, quadratisch wannenförmig, spitzwinkelig usw. sein. Es können aber auch reliefartige Erhöhungen auf der Manschetten-Innenseitenoberfläche angebracht sein, beispielsweise Wülste.

Anstelle von kontinuierlichen Verkörperungen mit Linearerstreckung können auch positive (reliefartige) wie negative (Ausnehmungen) diskontinuierliche "Linien"-Scharen vorgesehen sein, beispielsweise (positive) Noppen oder (negative) Grübchen. In allen Fällen gilt hinsichtlich der Dimensionierung des Koordinaten-Rasters, daß seine Maschenweite mindestens doppelt so groß ist wie die Quer-Abmessung der positiven oder negativen "Linien". Auch sind andere als in Fig. 1 dargestellte Raster - beispielsweise solche mit rautenförmigen Maschen - möglich.

Durch die Anbringung eines reliefartigen bzw. waffelartigen Rasters 6,7 in bzw. auf der Innenwandung 11 der Prothese 1 kann die gesamte Dauer des osteochirurgischen Eingriffs erheblich verkürzt werden. Der Operateur ist nämlich nunmehr in der Lage, durch wenige präoperative Einpaßversuche und zwar durch Heranführen des Total-Implantates an den Bereich der Gehörgangswand, den es durch einen chirurgischen Eingriff zu rekonstruieren gilt, und durch gleichzeitiges Abschätzen der anschließend durch Beischleifen zu entfernenden Flächenbereiche der Prothese, eine den individuellen Erfordernissen eines jeden Patienten entsprechende, optimal angepaßte Implantatform bereitzustellen. Bei der komplizierten Anatomie des äußeren und mittleren Gehörtraktes, bei den stark eingeschränkten Beobachtungsmöglichkeiten des Operateurs beim Einbringen des Implantates in den hinteren Gehörgangsbereich und bei den für normale chirurgische Verhältnisse kleinen Dimensionierungen des menschlichen Gehörganges bedeutet es einen erheblichen Fortschritt, wenn eine exakte Kontur des einzupflanzenden Daueimplantates durch nur einige Male auszuführendes "Maßnehmen" angefertigt werden kann.

Aus alledem ergibt sich, daß die Form und die Größe der Linienverkörperungen vorteilhafterweise derart ausgestaltet sein sollte, daß das zur besseren Ausleuchtung des Implantatortes beim präoperativen Einpassen vorhandene Beleuchtungslicht eine gewisse Schattenbildung an den Linienscharen des Rasters hervorruft. Durch eine derartige Schrägbeleuchtung wird der Reliefcharakter des Rasters hervorgehoben.

Versuche haben gezeigt daß ein Raster mit (negativen) Ausnehmungen vorteilhafter ist als ein solches mit (positiven) Wülsten oder Noppen, weil es bei letzterem gelegentlich vorkommen kann, daß bei der postoperativen Kontrolle etwaige Veränderungen zu Fehlinterpretationen Anlaß geben könnten.

Aus fertigungstechnischen Gründen, vor allem aber aus übergeordneten immunologischen Gesichtspunkten, bestehen die positiven Raster-Kennungen aus demselben Biomaterial wie die Prothese selbst. Auch im Falle der negativen Raster-Kennungen sind keine zusätzlichen, etwa andersfarbigen, optischen Konturverstärkungen vorgesehen.

Als Material kann bioaktive Glaskeramik zum Einsatz kommen, wie sie etwa in der DE-PS 23 26 100 ausführlich beschrieben ist. Andere geeignete Werkstoffe sind bioaktive Verbundmaterialien auf der Basis apatithaltiger Sinterprodukte, die aus den DE-PS 23 46 739 und DE-PS 24 34 979 bekannt sind. Des weiteren sind polymere bioaktive Verbundmaterialien verwendbar wie sie durch die DE-PS 25 01 683 geschützt sind. Schließlich ist es prinzipiell auch möglich, ein an sich bekanntes, aus bioinertem Material bestehendes Kernmaterial mit einer totalen oder partiellen Beschichtung aus bioaktivem Material zu versehen, wie es beispielsweise in der AT-PS 347 023 beschrieben ist.

In Fig. 3 ist in gestrichelter Form wiederum die in Fig. 1 dargestellte Manschette 1 gezeigt. In ausgezogenen Linien ist ein Teil 8 eines Manschetten-Abschnittes dargestellt. Darunter wird ein im wesentlichen senkrecht zur Manschetten-Achse 2 geführter peripherer Abschnittsteil verstanden. Natürlich sind - entsprechend der vorgesehenen Verwendung - auch diverse andere Formen von Manschetten-Abschnitten bztw. Manschetten-Ausschnitten von bzw. aus der als Raumform vorgegebenen Totalprothese 1 möglich.

In Fig. 3 ist lediglich ein Teil eines derartigen Manschetten-Abschnittes dargestellt. Dieser Teil 8 stellt ein Partialimplantat zur teilweisen Rekonstruktion des hinteren Teils der Gehörgangswand dar. Aus Gründen einer besseren Übersichtlichkeit ist hier das an sich auch vorhandene Innen-Raster weggelassen.

Der Einsatz des Biomaterials als Werkstoff für die er-findungsgemäße Prothese bietet gerade im Bereich der Mittelohrchirurgie besondere Vorteile, die in der DE-A-30 36 245.9 ausführlich beschrieben wurden.

Es ist bekannt, daß bioaktive Knochenersatzmaterialien - insbesondere Bioglas oder Bioglaskeramik - eine gewisse Oberflächenlöslichkeit aufweisen, welche vermutlich eine für das Zustandekommen des Knochen/Implantat-Verbundes notwendige Voraussetzung darstellt. Andererseits kann diese spezielle Eigenschaft unter besonders ungünstigen lokalanatomischen Bedingungen wie sie insbesondere bei der Implantation einer Prothese im Weichgewebelager bzw. bei Kontaktierung einer derartigen Prothese mit Weichgewebe gegeben sind dazu führen, daß die Langzeitstabilität des Implantates beeinträchtigt wird. Da die Eigenschaft die Bioaktivität durch besondere chemische Zusammensetzung des Implantatmaterials erzielt wird, ist ein solches Material für die Implantation in andere Gewebe nicht besonders vorteilhaft. So kann z.B. beobachtet werden daß die Implantation von bioaktivem Material stärkere empfindliche Reaktionen im Weichgewebe auslöst als ein bioinertes Material.

Da die erfindungsgemäße Gehörgangswand-Prothese jedenfalls im Bereich des oberen und unteren Manschettenrandes 9 bzw. 10 in direktem Kontakt mit knöchernem Gewebe kommt, werden diese Oberflächenbereiche nicht bioinaktiviert. Dagegen können die Innenwand 11 und/oder die Außenwand 12 und/oder die beiden Längsseiten 4 teilweise oder ganz eine bioinerte, unlösliche Schutzschicht aufweisen.

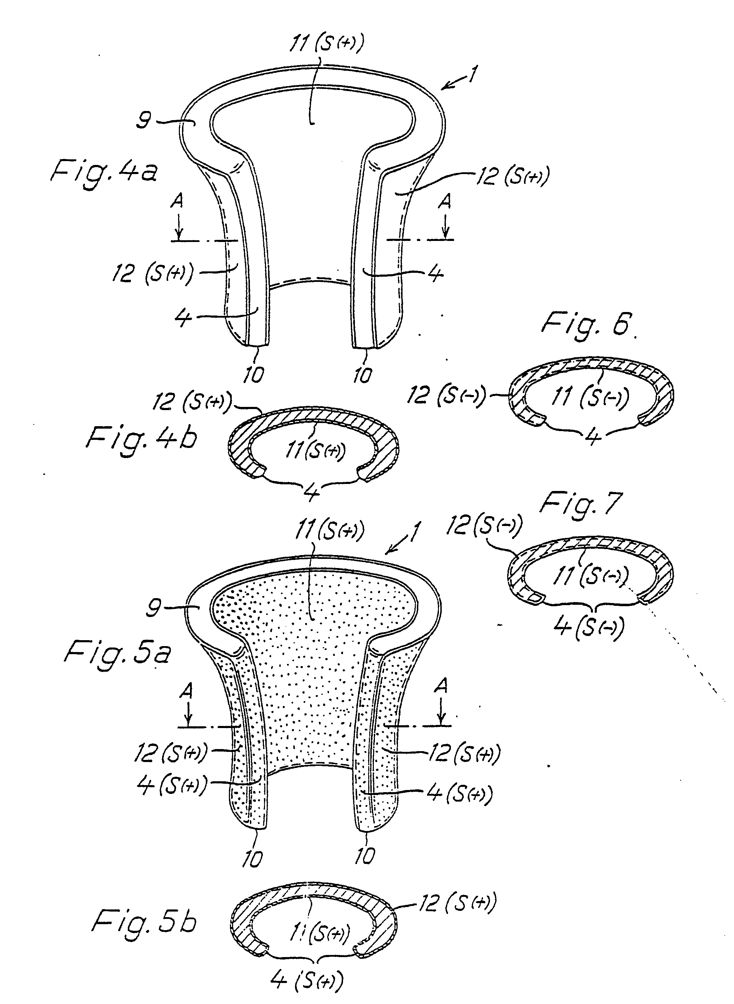

In Fig. 4a ist eine derartige Schicht - und zwar eine additive Schutzschicht S(+) - dargestellt. Sie bedeckt die Innenwand 11 (Kombinationsbezugszeichen: "11(S(+))" und die Außenwand 12 (Kombinationsbezugszeichen: "12(S(+))"). Da die Schutzschicht S(+) ihre inaktivierende Wirkung bereits bei sehr geringen Schichtdicken erzielt, wird durch die Aufbringung einer derartigen additiven Beschichtung die auf der Grundform vorhandene Rasterung optisch nicht "zugedeckt".

In den Fig. 5a und 5b wird eine additiv beschichtete Ausführungsform dargestellt, bei der außerdem die Längsseiten 4 der Manschette 1 bioaktiv gemacht wurden.

Die Fig. 6 zeigt einen Schnitt längs der gedachten Linie A-A bei einer Manschette, die in der Innen- und der Außenwand 11 und 12 eine geometrisch nicht auftragende, bioinerte, unlösliche, subtraktive Schicht S(-) aufweist (Kombinationsbezugszeichen: "11(S(-))" und "12(S(-))"). In Fig. 7 ist ein Beispiel gezeigt, bei dem zusätzlich auch die Längsseiten subtraktiv beschichtet wurden ("4(S(-))").

Beide - die additive und die subtraktive - Schutzschichten S(+) und S(-) sind hinsichtlich ihrer Funktionen gleichwirkend. Sie stellen gewissermaßen biochemische "Korrosions"-Schutzschichten dar, die komplette Transport-und-Durchlaß-Barrieren für jedweden Stoff-(Ionen)Austausch zwischen den chemischen Bestandteilen des Implantates und den Bestandteilen der physiologisch-biochemischen Körperflüssigkeiten gewährleisten.

Beispielsweise kann eine subtraktive Schutzschicht S(-) in vitro dadurch erhalten werden, daß bei einem aus bioglaskeramischem Voll-Material oder aus Bioglas-Material bestehendem Implantat diejenigen Oberflächen-Teilbereiche, die zwangsweise - also aufgrund anatomischer Gegebenheiten - oder vom Otochirurgen gewolltalso beispielsweise bei dem Auskleiden bestimmter Implantatbereiche mit Epithelgewebe - mit Weichgewebe in Dauerkontakt kommen, chemisch in der Weise vorbehandelt werden, daß wässrige saure Lösungen und/oder wässrige Salzlösungen in Normalitäten zwischen 0 001 und 0,1 die ursprüngliche bioglaskeramische Oberfläche attackieren, wobei Angriffs-(Auflösungs- bztw. Auslaugungs-) und Austausch-Reaktionen nebeneinander ablaufen mit der Folge, daß es zunächst zu einer Verarmung und schließlich zu einer vollständigen Zerstörung (Umwandlung) einer Phase - insbesondere der kristallinen Komponente(n) - des glaskeramischen Verbundsystems kommt. Zum chemischen Angriff können aber auch Basen und Puffersysteme verwendet werden, die in Abhängigkeit von ihrem konkreten Chemismus, ihrer Konzentration und ihrem $p_H$-Wert gezielt auf bestimmte Phasenkomponenten des Bioglaskeramik- "Verbundsystems" einwirken und so die gewünschte Eigenschaftsänderung der Implantatoberfläche bewirken.

Die nach dieser kombinierten chemischen Behandlung verbleibende Glaskeramik, die ihrer Apatitanteile beraubt ist, sei mit "Residual-Bioglaskeramik" bezeichnet. Sie ist bezüglich ihrer chemischen Eigenschaften unlöslich, porenfrei und stoppt jeglichen Ionentransport; sie ist bezüglich ihrer biochemisch-physiologischen Wirkung bioaktiv, also bio-"inert" und bezüglich ihrer mechanischen Eigenschaften abriebresistent und auf dem Prothesenkernmaterial festhaftend. Wie aus den Fig. 6 und 7 ersichtlich, wird durch die Erzeugung einer subtraktiven Schicht S(-) das Gesamtvolumen des Implantates nicht verändert. Es finden vielmehr chemische Austausch- bzw. Umwandlungsvorgänge in den Oberflächenbereichen statt die gewissermaßen "nach innen" gerichtet sind. Dagegen befinden sich die in den Fig. 4a - 5b dargestellten additiven Schichten S(+) auf der Implantatoberfläche, so daß das Gesamtvolumen der beschichteten Prothesen (Fig. 4a bztw. 5a) etwas vergrößert wurde.

Im Bedarfsfall kann die subtraktive Schicht S(-) zusätzlich auf thermischem Wege verdichtet bzw. versiegelt werden. Darüber hinaus ist im Bereich der bioinerten Beschichtung auch eine Silanisierungsschicht zusätzlich aufbringbar.

Mit der erfindungsgemäßen Prothese ist es also möglich, die unterschiedlichsten Teilbereiche bzw. den gesamten hinteren Trakt der menschlichen Gehörgangswand - ausgehend von einem vorhandenen Total-Implantat - in anatomisch korrekter Weise in kürzester Zeit dauerhaft prothetisch zu rekonstruieren.

**Patentansprüche**

1. Gehörganswand-Prothese in Form eines Hohlkörpers, dadurch gekennzeichnet, daß sie die Form einer nicht geschlossenen hohlzylindrischen Manschette (1) mit mindestens in ihren beidem Grundflächenbereichem vorhandenen unterschiedlichen Aufweitungen nach Art eines Hyperboloids aufweist, und daß sie aus bioaktivem Material besteht.

2. Gehörgangswand-Prothese nach Anspruch 1 dadurch gekennzeichnet, daß die Manschette einen ellipsenförmigen Querschnitt aufweist.

3. Gehörgangswaud-Prothese nach Anspruch 1 oder 2, dadurch gekennzeichnet, daß ihre Innenwandung (11) ein räumliches Koordinaten-Raster aufweist.

4. Gehörgangswand-Prothese nach Anspruch 3, dadurch gekennzeichnet daß das Raster aus zwei sich kreuzenden Scharen von Linien (6,7) besteht.

5. Gehörgangswand-Prothese nach Anspruch 4, dedurch gekennzeichnet, daß die eine der beiden Scharen aus einer Vielzahl von hyperbelförmigen, im wesentlichen in Richtung der Achse (2) der Manschette (1) verlaufenden Linien (6) und die andere der beiden Scharen aus einer Vielzahl von ellipsenförmigen, im wesentlichen senkrecht zur Achse (2) der Manschette (1) werlaufenden Linien (7) besteht.

6. Gehörgangswand-Prothese nach mindestens einem der Ansprüche 3 bis 5, dadurch gekennzeichnet, daß die Linien (6,7) des Koordinaten-Rasters als rillenförmige Vertiefungeu von beliebiger Querschnittsform - beispielsweise dreieckig, viereckig, abgerundet, usw. - ausgebildet sind.

7. Gehörgangswand-Prothese nach mindestens einem der Ansprüche 3 bis 5, dadurch gekennzeichnet, daß die Linien (6,7) des Koordinaten-Rasters als wulstartige Erhöhungen ausgebildet sind.

8. Gehörgamgswand-Prothese nach mindestens einem der Ansprüche 3 bis 7, dadurch gekennzeichnet, daß anstelle kontinuierlicher Linien (6,7) diskontinuierliche, periodisch angeordnete Raster, beispielsweise Grübchen oder Noppen, vorgesehen sind.

9. Gehörgangswand-Prothese nach mindestens einem der Ansprüche 1 bis 5 sowie 7 und 8, dadurchgekennzeichnet, daß die positiven Verkörperungen des Rasters aus demselben Material wie die Prothese selbst bestehen.

10. Gehörgangswand-Prothese nach mindestens einen der Ansprüche 3 bis 9, dadurch gekennzeichnet, daß die mittlere Maschenweite des Koordinaten-Rasters zwischen 1 und 2 mm beträgt.

11. Gehörgangswand-Prothese nach den Ansprüchen 1 und 2, dadurch gekennzeichnet, daß die Länge (4) des Manschetten-Ausschnittes (3) größer als dessen mittlere Peripher-Distanz (5) und daß die kürzeste aller Hauptachsen derjenigen Innen-Ellipsen, die durch Schnitte senkrecht zur Achse (2) der Manschette (1) erzeugt werden, länger als die mittlere Peripher-Distanz (5) des Manschetten-Ausschmittes (3) ist.

12. Gerhörgangswand-Prothese nach einen der Ansprüche 1, 2 und 11, dadurch gekennzeichnet, daß die Länge der Manschette (1) zu den beiden Mantel-Schnittstellen hin, an welchen der Manschetten-Ausschnitt (3) herausgetrennt ist, kontinuierlich größer wird und dort ihr Maximum erreicht, während ihr Minimum bei dem dem Ausschnitt (3) gegenüberliegenden Mantelbereich erreicht wird.

13. Gehörgangswand-Prothese nach einem der vorherigen Ansprüche, dadurch gekennzeichnet, daß die Wanddicke der Manschette (1) zwischen 0,5 und 2,0 mm, die mittlere Länge der Manschette (1) zwischen 12 und 25 mm, die mittlere Peripher-Distanz (5) des Manschetten-Ausschnittes (3) zwischen 7 und 14 mm, die mittlere Länge der Ellipsen-Hauptachsen zwischen 10 und 17 mm, die mittlere Länge der Ellipsen-Nebenachsen zwischen 5 und 8 mm betragt und das positive bzw. negative Raster eine mittlere Höhe bzw. Tiefe zwischen 0,2 und 1,0 mm aufweist.

14. Gehörgangswand-Prothese nach mindestens einem der vorherigen Ansprüche, dadurch gekennzeichnet, daß das bioaktive Material ein Verbundmaterial ist.

15. Gehörgangswand-Prothese nach mindestens einem der vorherigen Ansprüche, dadurch gekennzeichnet, daß das bioaktive Material aus apatithaltigen Sinterprodukten besteht.

16. Gehörgangswand-Prothese nach mindestens einem der vorherigen Ansprüche, dadurch gekennzeichnet, daß das bioaktive Material aus Glaskeramik besteht.

17. Gehörgangswand-Prothese nach mindestens einem der vorherigen Ansprüche, dadurch gekennzeichnet, daß sie aus einem Kernmaterial mit einer Beschichtung aus bioaktivem Material besteht.

18. Gehörgangswand-Prothese nach mindestens einem der vorherigen Ansprüche, dadurch gekennzeichnet, daß sie lediglich aus einem Teil der Gesamtmanschette (1), insbesondere aus einem Manschettenmantel-Abschnitt (8) oder einem Teil davon, besteht.

19. Gehörgangswand-Prothese nach mindestens einem der vorherigen Ansprüche, dadurch gekennzeichnet, daß sie zusätzlich in mindestens einem Oberflächen-Teilbereich unlösliches bioinertes Material enthält.

20. Gehörgangswand-Prothese nach Anspruch 19, dadurch gekennzeichnet, daß sie partiell eine Beschichtung aus unlöslichem bioinerten Material aufweist.

21. Gehörgangswand-Prothese nach Amspruch 20, dadurch gekennzeichnet, daß die Beschichtung aus mindesteus einer additiv aufgebrachten Schutzschicht (S(+)) mit einer Dicke zwischen 0,25 und 10 µm besteht.

22. Gehörgangswand-Prothese nach Anspruch 20, dadurch gekennzeichnet, daß die Beschichtung aus mindestens einer subtraktiv erzeugten Konversionsschicht (S(-)) mit einer Dicke zwischen 0,25 und 5 µm besteht.

23. Gehörgangswand-Prothese nach den Ansprüchen 20 bis 22, dadurch gekennzeichnet, daß die Beschichtung aus mindestens einer prothesenkernseitig erzeugten Konversionsschicht (S(-)) und mindestens einer auf dieser aufliegenden, additiv aufgebrachten Schutzschicht (S(+)) besteht.

24. Gehörgangswand-Prothese nach mindestens einem der nsprüche 19 bis 23, dadurch gekennzeichnet, daß zumindest die Innenwand (11) und/oder die Außenwand (12) der Manschette (1) aus unlöslichem bioinerten Material besteht.

25. Gehörgangswand-Prothese nach Anspruch 24, dadurch gekennzeichnet, daß zusätzlich die Längsseiten (4) der Manschette (1) aus unlöslichem bioinerten Material bestehen.

26. Gehörgangswand-Prothese nach mindestens einem der nsprüche 19 bis 25, dadurch gekennzeichnet,daß das bioinerte Material bzw. die bioinerte Schicht (S(+)) bzw. (S(−)) aus mindestens einer der folgenden Substanzen besteht:

a) Metalle, wie Gold, Platin, Titan, sowie Metallegierungen;

b) Kohlenstoff in geeigneten Modifikationen, wie pyrolytischer Kohlenstoff (Graphit); Kohlenstoffverbindungen, wie Siliziumkarbid (SiC); Titankarbid (TiC), Borkarbid ($B_4C$);

c) Sonderkeramische Werkstoffe, wie hexagonales Bornitrid (BN), Titannitrid (TiN), Siliciumnitrid ($Si_3N_4$);

d) teilkristaline anorganische Verbundsgsteme, wie Emails;

e) anorganische Einkomponenten- (z.B. Kieselglas) oder Mehrkomponenten-Gläser;

f) Oxide, wie Titandioxid ($TiO_2$), Zirkondioxid ($ZrO_2$) und Aluminiumoxid-($Al_2O_3$).

27. Gehörgangswand-Prothese nach mindestens einem der Ansprüche 19 bis 26). dadurch gekennzeichnet, daß das bioinerte Material bzw. die bioinerte Schicht (S(-)) aus einer(m) apatitfreien Residual-Bioglaskeramik bzw. -Bioglas besteht, welche(s) gegebenenfalls zusätzlich eine Silan-Schicht aufweist.

28. Verfahren zur Herstellung einer Prothese nach mindestens einem der Ansprüche 19 bis 27, dadurch gekennzeichnet, daß die zu behandelnden Teilbereiche der Manschette (1) unter gezielter Stoffzufuhr einer Oberflächen-Nachbehandlung zur Erzeugung mindestens einer additiv aufgebrachten, dauerhaft festsitzenden, in vivo als biochemische Sperrschicht wirkenden, bioinerten Schutzschicht (S(+)) unterworfen werden.

29. Verfahren nach mindestens einem der AnSprüche 19 bis 27, dadurch gekennzeich net, daß die zu behandelnden Tailbereiche der Manschette (1) unter gezielter Stoffabfuhr bzw. Stoffaustausch einer chemischen Nachbehandlung zur Erzeugung mindestens einer subtraktiv erhaltenen, permanent festhaftenden, in vivo als biochemische Sperrschicht wirkenden, bioinerten Konversions(Auslaugungs-)Schicht (S(-)) unterworfen werden.

30. Verfahren nach Anspruch 28, dadurch gekennzeichnet, daß die Schutzschicht (S(+)) durch mindestens einen der folgenden Schritte Vorzugsweise aufgebracht wird:

a) Galvanisieren oder Bedampfen;

b) Aufsputtern;

c) Abscheiden aus organischen Lösungen oder Aufdampfen im Vakuum;

d) Tauchen, Sprühen oder Aufstreuen mit nachfolgender thermischer Behandlung;

e) Eintauchen in Wasserglas mit anschließendem Aufheizen auf ca. 400°C bzw. Tauchglasieren aus ein- oder mehrkomponentigen Schwelzgemengen;

f) Simultan-Aufdampfen oder Aufdampfen der Metalle mit nachfolgender Oxidierungsbehandlung.

31. Verfahren nach Anspruch 29, dadurch gekennzeichnet, daß die zu beschichtenden Oberflächen-Teilbereiche der Manschette (1) mit wässrigen, sauren Lösungen oder mit wässrigen Salzlösungen mit Normalitäten zwischen 0,001 und 0,1 zwischen 5 Minuten und 3 Stunden bei Temperaturen Zwichen 20 bis 100° C behandelt werden.

32. Verfahren nach Anspruch 31, dadurch gekennzeichnet, daß als wässrige, saure Lösung 0,1 - 0,001 normale Salzsäure (HC1) verwendet wird.

33. Verfahren nach Anspruch 31, dadurch gekennzeichnet, daß als wässrige Salzlösung 0,001 bis 0,25 normale Standard-Azetat-Puffer-Lösung Verwendet wird.

34. Verfahren nach Anspruch 29, dadurch gekennzeichnet, daß die beschichteten Teile (3) der Manschette (11,12; 4) anschließend einer thermischen Versiegelungs- und/oder Silanisierungsbehandlung unterworfen werden.

## Claims

1. Prosthesis, in the shape of a hollow body, of the wall of the acouatic duct, characterised thereby, that it displays the shape of a not closed, hollow cylindricel sleeve (1) with different enlargements in the manner of a hyperboloid, which are present in at least both its base surfaces, and that it consists of bio-active material.

2. Prosthesis of the wall of the acoustic duct according to claim 1, characterised thereby, that the slseve displays an elliptically shaped cross-section.

3. Prosthesis of the wall of the acoustic duct according to claim 1 or 2, characterised thereby, that its inside wall (11) displays a three-dimensional co-ordinate raster.

4. Prosthesis of the wall of the acoustic duct according to claim 3, characterised thereby, that the raster consists of two mutually crossing families of lines (6, 7).

5. Prosthesis of the wall of the acoustic duct according to claim 4, characterised thereby, that the one of both the families consists of a plurality of hyperbolically shaped lines (6) extending substantially in direction of the axis (2) of the sleeve (1) and the other one of both the families coneists of a plurality of elliptically shaped lines (7) extending substantially perpendicularly to the axis (2) of the sleeve (1).

6. Prosthesis of the wall of the acoustic duct according to at least one of the claims 3 to 5, characterised thereby, that the lines (6, 7) of the co-ordinate raster are formed as fluted depressions of any desired cross-sectional shape, for example triangular, quadrangular, rounded off and so forth.

7. Prosthesis of the wall of the acoustic duct according to at least one of the claims 3 to 5, characterised thereby, that the lines (6, 7) of the co-ordinate raster are formed as beadlike protrusions.

8. Prosthesis of the wall of the acoustic duct according to at least one of the claims 3 to 7, characterised thereby, that discontinuous, periodically arranged rasters, for example dimPles or nubs, are provided in place of continuous lines (6, 7).

9. Prosthesis of the wall of the acoustic duct according to at least one of the claims 1 to 5 as well as 7 and 8, characterised thereby, that the positive embodiments of the raster consist of the same material as the prosthesis itself.

10. Prosthesis of the wall of the acoustic duct according to at least one of the claims 3 to 9, charactarised thereby, that the mean mesh width of the co-ordinate raster amounts to between 1 and 2 millimetres.

11. Prosthesis of the wall of the acoustic duct according to the claims 1 and 2, characterised thereby, that the length (4) of the sleeve cut-out (3) is greater than its mean peripheral distance (5) and thst the shortest of all principal axes of those internal ellipses, which are generated by sections perpendicular to the axis (2) of the sleeve (1), is longer than the mean peripheral distance (5) of the sleeve cut-out (3).

12. Prosthesis of the wall of the acoustic duct according to one of the claims 1, 2 and 11, characterised thereby, that the length of the sleeve cut-out (3) becomes continuously greater towards both the envelope section places, at which the sleeve cut-out (3) is severed out, and there reaches its msximum, whilst its minimum is reached at the envelope region lying opposite the cut-out (3).

13. Prosthesis of the wall of the acoustic duct according to one of the preceding claims, characterised thereby, that the wall thickness of the sleeve (1) amounts to between 0.5 and 2 millimetres, the mean length of the sleeve (1) to betwaen 12 and 25 millimetres, the mean peripheral distance (5) of the sleeve cut-out (3) to between 7 and 14 millimetres, the mean length of the principal sxes of the ellipse to between 10 and 17 millimetres and the mean length of the secondary axes of the ellipse to between 5 and 8 millimetres and the positive or negative raster displays a mean height or depth of between 0.2 and 1.0 millimetres.

14. Prosthesis of the wall of the acoustic duct according to at least one of the preceding claims, characterised thereby, that the bio-active material is a compound material.

15. Prosthesis of the wall of the acoustic duct according to at least one of the preceding claims, characterised thereby, that the bio-active material consists of sintered products containing apatite.

16. Prosthesis of the wall of the acoustic duct according to at least one of the preceding claims, characterised thereby, that the bio-active material consists of glass-ceramic material.

17. Prosthesis of the wall of the acoustic duct according to at least one of the preceding claims, characterised thereby, that it consists of a core material with a coating of bio-active material.

18. Prosthesis of the wall of the acoustic duct according to at least one of the preceding claims, characterised thereby, that it consists merely of a part of the entire sleeve (3), in particular of a sleeve enuelope portion (8) or a part thereof.

19. Prosthesis of the wall of the acoustic duct according to at least one of the preceding claims, characterised thereby, that it additionally contains insoluble bio-inert material in at least a partial surface region.

20. Prosthesis of the wall of the acoustic duct according to claim 19, characterised thereby, that it in part displaya a coating of insoluble bio-inert material.

21. Prosthesis of the wall of the acoustic duct according to claim 20, characterised thereby, that the coating consista of at least one additively applied protective layer (S( +)) of a thickness of between 0.25 and 10 micrometres.

22. Prosthesis of the wall of the acoustic duct according to claim 20, characterised thereby, that the coating conaists of at least one subtractively produced conversion layer (S( -)) of a thickness of between 0.25 and 5 micrometres.

23. Prosthesis of the wall of the acoustic duct according to the claims 20 to 22, characterised thereby, that the coating consists of at least one conversion layer (S( -)) produced at the side of the core of the prosthesis and at least one additively applied protective layer (S( +)) resting on this.

24. Prosthesis of the wall of the acoustic duct according to at least one of the claims 19 to 23, characterised thereby, that at least the inside wall (11) and/or the outside wall (12) of the sleeve (1) consists of insoluble bio-inert material.

25. Prosthesis of the wall of the acoustic duct according to claim 24, characterised thereby, that the longitudinal sides (4) of the sleeve (3) consist of insoluble bio-inert material.

26. Prosthesis of the wall of the acoustic duct according to at least one of the claims 19 to 25, characterised thereby, that the bio-inert material or the bio-inert layer (S( +)) or (S( -)) consists of at least one of the following substances:

a) metals such as gold, platinum, titanium as well as metal alloys,

b) carbon in suitable modifications, such as pyrolytic carbon (graphite), carbon compounds euch as silicon carbide (SiC), titanium carbide (TiC) and boron carbide ($B_4C$),

c) special ceramic materials such as hexagonal boron nitride (BN), titanium nitride (TiN) and silicon nitride ($Si_3N_4$),

d) partially crystalline inorganic compound systems such as enamels,

e) inorganic single-component glasses (for example flint glass) or multi-component glasses, and

f) oxides such as titenium dioxide ($TiO_2$), zirconium dioxide ($ZrO_2$) and aluminium oxide ($Al_2O_3$).

27. Prosthesis of the wall of the acoustic duct according to at least one of the claims 19 to 26, characterised thereby, that the bio-inert material of the bio-inert layer (S( -)) consists of a residual bioglass ceramic material or a residual bioceramic material or a bioglass, which is apatite-free and in a given case additionally displays a silane layer.

28. Process for the manufacture of a prosthesis according to at least one of the claims 19 to 27, characterised thereby, that the partial regions of the sleeve (1), which are to be treated, are subjected under pinpointed substance supply to a surface-finishing treatment for the generation of at least one additively applisd, permanently adhering, inorganic bio-inert protsctive layer (S( +)), which in vivo acts as biochemical barrier layer.

29. Process according to at least one of the claims 19 to 27, characterised thereby, that the partial regions of the sleeve (1), which are to be treated, are subjected under pinpointed substance removal or substance exchange to a chemical finishing treatment for the generation of at least one subtractively obtained, permanently adhering bio-inert conversion (leaching) Layer (S( -)), which in vivo acts as biochemical barrier layer.

30. Process according to claim 28, characterised thereby, that the protective layer (S( +)) is preferably applied through at least one of the following steps:

a) galvanising or vapour-depositing,

b) sputtering,

c) precipitating out of organic solutions or vapourising under vacuum,

d) dipping, spraying or scattering-on with subsequent thermal treatment,

e) immersion in water glass with subsequent heating to about 400°C or immersion-glazing from single-component or multi-component smelt batches and

f) simultaneous evaporation or evaporation of ths mstals with subsequent oxidising treatment.

31. Process according to claim 29, characterised thereby, that the partial surface regions of the sleeve (1), which are to be coated, are treated with aqueous acid solutions or with aqusous salt solutions of molecular concentrations between 0.001 and 0.1 for between 5 minutes and 3 hours at temperatures between 20 to 100°C.

32. Process according to claim 31, characterised thereby, that hydrochloric acid (HCl) of a molecular concentration between 0.1 and 0.001 is used as aqueous acid solution.

33. Process according to claim 31, characterised thereby, that standard acetate buffar solution of a molecular concentration between 0.001 and 0.25 is used as aqueous salt solution.

34. Process according to claim 29, characterised thereby, that the coated parts of the prosthesis are subsequently subjected to a thermal sealing treatment and/or silanising treatment.

## Revendications

1. Prothèse de la paroi du conduit auditif sous la forme d'un corps creux caractérisée en ce qu'elle a la forme d'une manchette en cylindre creux, non fermé (1) avec au moins des élargissements différents dans ses deux zones de surface de base, du type hyperboloïde, et en ce qu'elle se compose d'une matière bioactive.

2. Prothèse de la paroi du conduit auditif selon la revendication 1 caractérisée en ce que la manchette présente une coupe en forme d'ellipse.

3. Prothèse de la paroi du conduit auditif selon la revendication 1 ou 2 caractérisée en ce que sa paroi interne (11) présente un réseau de coordonnées dans l'espace.

4. Prothèse de la paroi du conduit auditif selon la revendication 3 caractérisée en ce que le réseau se compose de deux groupes de lignes (6,7) se croisant.

5. Prothèse de la paroi du conduit auditif selon la revendication 4 caractérisée en ce que le premier des deux groupes se compose d'un grand nombre de lignes (6) en forme d'hyperbole, essentiellement dans la direction de l'axe (2) de la manchette (1) et l'autre des deux groupes se compose d'un grand nombre de lignes (7) en forme d'ellipse, essentiellement perpendiculaires à l'axe (2) de la manchette 1.

6. Prothèse de la paroi du conduit auditif selon au moins l'une des revendications 3 à 5 caractérisée en ce que les lignes (6,7) du réseau de coordonnées sont configurées en enfoncements enforme de rainures de forme en coupe souhaitée, par exemple rectangulaire,quadrangulaire, arrondie etc.

7. Prothèse de la paroi du conduit auditif selon au moins l'une des revendications 3 à 5 caractérisée en ce que les lignes (6,7) du réseau de coordonnées ont la forme de surélévations en bourrelet

8. Prothèse de la paroi du conduit auditif selon au moins l'une des revendications 3 à 7 caractérisée en ce qu'au lieu de lignes continues (6,7), sont prévus des réseaux discontinus et disposés de manière périodique, par exemple des piqures ou des noppes.

9. Prothèse de la paroi du conduit auditif selon au moins l'une des revendications 1 à 5 ainsi que 7 et 8 caractérisée en ce que les représentations positives du réseau se composent du même matériau que la prothèse elle-même.

10. Prothèse de la paroi du conduit auditif selon au moins l'une des revendications 3 à 9 caractérisée en ce que la largeur moyenne de la maille du réseau de coordonnées est comprise entre 1 et 2 mm.

11. Prothèse de la paroi du conduit auditif selon les revendications 1 et 2 caractérisée en ce que la longueur (4) de la coupe (3) de la manchette est plus grande que sa distance périphérique moyenne (5) et en ce que le plus court de tous les axes principaux des ellipses internes, que l'on produit par des coupes perpendiculaires à l'axe (2) de la manchette (1), est plus long que la distance périphérique moyenne (5) de la coupe (3) de la manchette.

12. Prothèse de la paroi du conduit auditif selon l'une des revendications 1, 2 et 11 caractérisée en ce que la longueur de la manchette (1) jusqu'aux deux points d'intersection de coupe de l'enveloppe, où est séparée la coupe (3) de la manchette, augmente continuellement et y atteint son maximum tandis que son minimum est atteint dans la zone d'enveloppe face à la coupe (3).

13. Prothèse de la paroi du conduit auditif selon l'une quelconque des revendications précédentes caractérisée en ce que l'épaisseur de paroi de la manchette (1) est comprise entre 0,5 et 2,0 mm, la longueur moyenne de la manchette (1) est comprise entre 12 et 25 mm, la distance periphérique moyenne (5) de la coupe (3) de la manchette est comprise entre 7 et 14 mm, la longueur moyenne de l'axe principal de l'ellipse est comprise entre 10 et 17 mm, la longueur moyenne de l'axe auxiliaire de l'ellipse est comprise entre 5 et 8 mm et le réseau positif ou respectivement négatif présente une hauteur ou respectivement profondeur moyenne comprise entre 0,2 et 1,0 mm.

14. Prothèse de la paroi du conduit auditif selon au moins l'une des revendications précédentes caractérisée en ce que la matière bioactive est un matériau de jonction.

15. Prothèse de la paroi du conduit auditif selon au moins l'une des revendications précédentes caractérisée en ce que la matière bioactive se compose de produits frittés contenant de l'apatite.

16. Prothése de la paroi du conduit auditif selon au moins l'une des revendications précédentes caractérisée en ce que la matière bioactive se compose de vitrocéramique.

17. Prothèse de la paroi du conduit auditif selon au moins l'une des revendications précédentes caractérisée en ce qu'elle se compose d'un matériau de noyau avec une enduction d'une matiére bioactive.

18. Prothése de la pàroi du conduit auditif selon au moins l'une des revendications précédentes caractérisée en ce qu'elle ne se compose que d'une partie de la totalité de la manchette (1), en particulier d'un segment (8) de l'enveloppe de la manchette ou bien d'une partie de celui-ci.

19. Prothése de la paroi du conduit auditif selon au moins l'une des revendications précédentes caractérisée en ce qu'elle contient, de plus, dans au moins une zone partielle de sa surface, une matiére bioinerte insoluble.

20. Prothése de la paroi du conduit auditif selon la revendication 19 caractérisée en ce qu'elle présente partiellement une enduction d'une matiére bioinerte insoluble.

21. Prothése de la paroi du conduit auditif selon la revendication 20 caractérisée en ce que l'enduction se compose d'au moins une couche de protection (S(+))produite par addition ayant une épaisseur comprise entre 0,25 et 10 µm.

22. Prothése de la paroi du conduit auditif selon la revendication 20 caractérisée en ce que l'enduction se compose d'au moins une couche de conversion (S(-)) produite par soustraction ayant une épaisseur comprise entre 0,25 et 5 µm.

23. prothése de la paroi du conduit auditif selon les revendications 20 à 22 caractérisée en ce que l'enduction se compose d'au moins une couche de conversion (S(-)) produite du côté noyau de la prothése et d'au moins une couche de protection formée par addition S(+)) reposant sur celle-ci.

24. Prothése de la paroi du conduit auditif selon au moins l'une des revendications 19 à 23 caractérisée en ce qu'au moins la paroi interne (11) et/ou la paroi externe (12) de la manchette (1) se compose d'une matière bioinerte insoluble.

25. Prothése de la paroi du conduit auditif selon la revendication 24 caractérisée en ce que, de plus, les côtés longitudinaux (4) de la manchette (1) se composent d'une matière bioninerte insoluble.

26. Prothése de la paroi du conduit auditif selon au moins l'une des revendications 19 à 25 caractérisée en ce que la matière bioinerte ou respectivement la couche bioinerte (S(+)) ou respectivement (S(-)) se compose d'au moins l'une des subsrances suivantes:

a) Métaux comme or, platine, titane, ainsi qu'alliages de métaux;

b) Carbone sous ses modifications appropriées comme du carbone pyrolytique (graphite); des composés de carbone. comme du carbure de silicium (SiC), du carbure de titane (TiC), du carbure de bore ($B_4C$);

c) Matières céramiques spéciales comme du nitrure de bore hexagonal (BN), du nitrure de titane (TiN), du niture de silicium ($Si_3N_4$);

d) Systèmes composés anorganiques partiellement cristallins comme des émaux;

e) Verres anorganiques à un composant (par exemple quartz fondu) ou à plusieurs composants;

f) Oxydes comme le bioxyde de titane ($TiO_2$),le bioxyde de zirconium ($ZrO_2$) et l'oxyde d'aluminium ($Al_2O_3$)

27. Prothése de la paroi du conduit auditif selonau moins l'une des revendications 19 à 26, caractérisée en ce que la matiére bioinerte ou respectivement la couche bioinerte (S(−)) se compose d'une biovitrocéramique résiduelle ou respectivement bioverre sans apatite, qui le cas échéant présente de plus une couche de silane.

28. Procédé de fabrication d'une prothése selon au moins l'une des revendications 19 à 27 caractérisé en ce que les zones partielles à traiter de la manchette (1) sont soumises, avec apport voulu de matière, à un post-traitement de surface pour la production d'au moins une couche bioinerte de protecrion (S(+)) produite par addition, fixée de manière. durable, agissant in vivo comme couche biochimique de barrage.

29. Procédé selon au moins l'une des revendications 19 à 27 caractérisé en ce que les zones partielles à traiter de la manchette (1) sont soumises, avec retrait voulu de matiére ou respectivement échange,de matière, à un post-traitement chimique pour la production d'au moins une couche (s(-)) de conversion (extraction)bioinerte obtenue par soustraction, fixée en permanence, agissant in vivo comme couche biochimique de barrage.

30. Procédé selon la revendication 28 caractérisé en ce que la couche de protection (S(+)) est produite avantageusement par au moins l'une des étapes suivantes:

a) Galvanisation ou métallisation au vide;

b) Crachement;

c) Séparation de solutions organiques ou métallisation sous vide;

d) Immersion, aspersion ou soupoudrage avec ensuite traitement thermique;

e) Immersion dans du verre soluble avec ensuite chauffage à environ 400°C ou respectivement glaçage par immersion dans des masses fondues à un ou plusieurs composants;

f) Métallisation simultanée sous vide ou métallisation des métaux avec ensuite traitement d'oxydation.

31. Procédé selon la revendication 29 caractérisé en ce que les zones partielles de surface à enduiie de la manchette (1) sont traitées avec des solutions acides aqueuses ou avec des solutions aqueuses de sel ayant des normalités entre 0,001 et 0,1 entre 5 minutes et 3 heures à des températures entre 20 et 100°C.

32. Procédé selon la revendication 31 caractérisé en ce qu'on utilise, comme solution acide aqueuse, des sels acides (HCl) normaux à 0,1-0,001.

33. Procédé selon la revendication 31 caractérisé en ce qu'on utilise, comme solution aqueuse de sel, des solutions standards de tampon d'acétate normales à 0,001 à 0,25.

34. Procédé selon la revendication 29 caractérisé en ce que les parties enduites (3) de la manchette (11,12;4) sont finalement soumises à un traitement thermique de vitrification et/ou de silanisation.

0 064 277

*Fig. 1*

*Fig. 3*

*Fig. 2a*

*Fig. 2b*

$F_{min}$

1

Fig. 4a

Fig. 4b

Fig. 5a

Fig. 5b

Fig. 6

Fig. 7